# EUROPEAN PATENT SPECIFICATION

(11) **EP 3 946 558 B1**
(45) Date of publication and mention of the grant of the patent: **13.03.2024**
(21) Application number: 20784158.6
(22) Date of filing: 03.04.2020
(51) Int. Cl.: A61N 1/05, A61N 1/04, A61N 1/36, A61N 1/372

(54) **STIMULATING DEVICE INCLUDING AN ELECTRODE ARRAY**
REIZVORRICHTUNG MIT ELEKTRODENANORDNUNG
DISPOSITIF DE STIMULATION COMPRENANT UN RÉSEAU D'ÉLECTRODES

(30) Priority: 05.04.2019 US 201962830347 P
(43) Date of publication of application: 09.02.2022
(73) Proprietor: Memstim, LLC, Louisville KY 40202-2654 (US)
(72) Inventor: JOHNSON, Angelique Candace, Louisville, Kentucky 40202-2654 (US); STROTMAN, Lindsay Nicole, Louisville, Kentucky 40202-2654 (US)
(74) Representative: LKGlobal UK Ltd.
(86) International application number: PCT/US2020/026555
(87) International publication number: WO 2020/206235

(56) References cited:
- WO-A2-2018/098046
- US-A1- 2003 233 133
- US-A1- 2003 233 133
- US-A1- 2012 157 804
- US-A1- 2015 335 876
- US-A1- 2017 245 772

## Description

### CROSS-REFERENCE TO RELATED APPLICATION

This application claims the benefit of U.S. Provisional Application No. 62/830,347, filed April 5, 2019.

### FIELD OF THE DISCLOSURE

The present disclosure relates generally to a stimulating device including an electrode array.

### BACKGROUND

Neuroprosthetic devices are stimulating devices often used for treating neurological diseases and disorders such as Parkinson's disease, deafness, epilepsy, chronic pain, paralysis, and blindness. One example of a neuroprosthetic device is a cochlear implant, a medical device implanted into the cochlea of an organism. The cochlear implant includes a stimulator that generates electrical signals and an electrode array that provides the electrical signals to nerve fibers in the cochlea to improve hearing. An electrical connection is usually established between the stimulator and the electrode array with connector or feedthrough pins connected to a wire bundle welded to the stimulator and to the electrodes of the electrode array. Although suitable for establishing the electrical connection, welding is typically a complex manufacturing process. The wire bundle is prone to fracture, leading to higher numbers of revision surgeries. Additionally, the wire bundle is also difficult to assemble, which limits miniaturization of the stimulator and/or the electrode array. WO 2018/098046 A2 discloses an apparatus for providing cognitive or physical therapy to patients, having a signal detector for detecting biometric electrical signals, and where a microprocessor controls a signal detector, a signal generator, and an electrode multiplex circuit.

The present disclosure is aimed at solving these issues.

### SUMMARY

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

Embodiments of a stimulating device are provided. The stimulating device comprises a stimulator for generating electrical signals; an electrode array for transmitting the electrical signals to a target and the electrode array including a substrate defining an aperture and at least one electrode supported by the substrate; a connector extending from the stimulator and the connector disposed at least partially in the aperture of the substrate and defining a space between the connector and the substrate, with the connector configured to establish an electrical connection between the stimulator and the electrode array; and a conductive elastic ink disposed at least partially within the space to secure the connector within the aperture.

Embodiments of an electrode array are also provided. The electrode array comprises a substrate; and at least one electrode supported by the substrate, wherein the substrate defines an aperture for receiving a connector for establishing an electrical connection between a stimulator and the electrode array.

### BRIEF DESCRIPTION OF THE DRAWINGS

Advantages of the present invention will be readily appreciated, as the same becomes better understood by reference to the following detailed description, when considered in connection with the accompanying drawings.
Figure 1 is a semi-schematic, partial cross-sectional perspective view of a portion of an auditory system of an organism and a stimulating device implanted into the cochlea of the organism.
Figure 2 is a schematic perspective view of a segment of an electrode array for the stimulating device.
Figure 3 is an exploded, semi-schematic perspective view of the stimulating device according to an embodiment of the present disclosure.
Figure 4 is a cross-sectional view of a segment of the stimulating device taken along line 4-4 of Figure 3.
Figure 5 is a cross-sectional view of a segment of the of the stimulating device according to another embodiment of the present disclosure.
Figure 6 is a cross-sectional view of a segment of the stimulating device according to yet another embodiment of the present disclosure.

### DETAILED DESCRIPTION

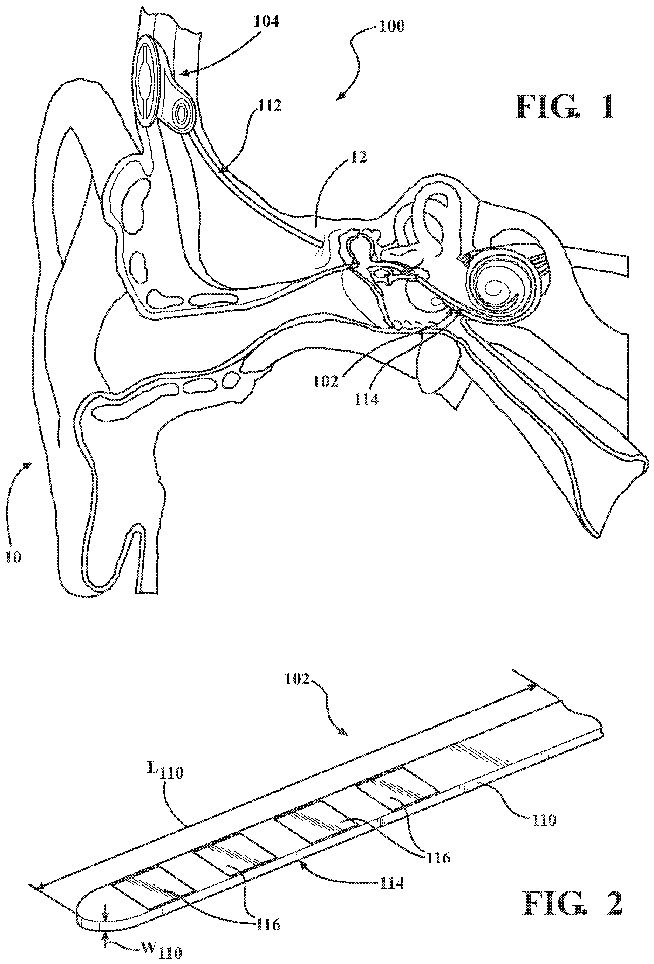

Referring now to the figures, where like numerals indicate like or corresponding parts throughout the several views, embodiments of a stimulating device and an electrode array 102 for the stimulating device are shown throughout the figures and described in detail below. In the embodiments described below, the stimulating device is a neuroprosthetic device adapted to supplant or supplement the input and/or output of the nervous system of an organism (such as an animal or human being). Neuroprosthetic devices are often used for treating various neurological diseases and disorders. Non-limiting examples of neuroprosthetic devices include deep brain stimulators, spinal cord stimulators, retinal prostheses and cochlear prostheses. For example, and as shown in Figure 1, the stimulating device may be a cochlear prosthesis 100, a device implantable into the cochlea 12 of an organism 10 for treating hearing loss. Due, at least in part, to the presence of a flexible material, such as silicone, in the individual material layers, the electrode array 102 of the stimulating device is flexible and elastic and can be readily implanted into small, curved spaces, such as the cochlea 12 of the organism 10. It should be appreciated that the stimulating device is not limited to cochlear prostheses. Applications outside of medical devices is also contemplated.

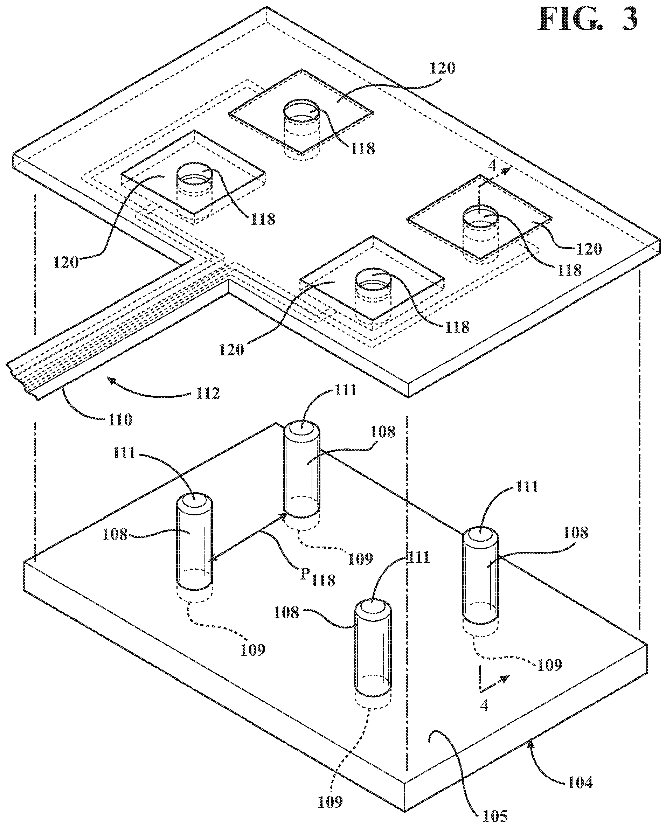

With continued reference to Figure 1, the stimulating device includes a stimulator 104 for generating electrical signals and the electrode array 102 for transmitting the electrical signals to a target, such as the cochlea 12 of the organism 10. The stimulating device further includes a connector 108, such as a connector pin or feedthrough pin schematically represented in Figures 3-6, extending from the stimulator 104. As described in detail below, the connector 108 is received and secured within an aperture 118 defined in a substrate 110 of the electrode array 102 to establish an electrical connection between the electrode array 102 and the stimulator 104.

As used herein, the stimulator 104 is or includes any device (having any suitable design or configuration) that operates to stimulate or produce a stimulus. For example, the stimulator 104 is a device that operates to generate electrical signals. In medical applications, the signals are used as a stimulus for treating various medical diseases or disorders. For example, the signals generated by the stimulator 104 may be used to stimulate nerves of the organism 10 to mask pain, to treat hearing loss, to control the function of organs, etc. Also, for medical applications, the stimulator 104 may or may not be implantable in the organism 10. In instances where the stimulator 104 is not implantable into the organism 10 (such as shown in Figure 1), the stimulator 104 may be referred to as an external device.

The electrode array 102 is electrically connected to the stimulator 104 (via the connector 108 as mentioned above) and, in medical applications, is typically implanted into the organism 10. The electrode array 102 is desirably formed from biocompatible materials, i.e., materials that are friendly and unharmful to living tissue. The electrode array 102 includes a configuration or array of electrodes 116 adapted to pass or transmit the electrical signals generated by the stimulator 104 to the target (such as, for example, a nerve or neuron in the cochlea 12 of the organism 10). In an alternative embodiment, a single electrode 116 could be used to pass electrical signals to the target. In this alternative embodiment, the electrode array 102 would be simply an electrode.

Figure 2 is a schematic representation of the stimulating device (e.g., the prosthesis 100) including the electrode array 102. With reference to Figures 1-3, the electrode array 102 has an interconnect region 112 and an electrode region 114. The interconnect region 112 allows for electrical connection of the electrode array 102 to the stimulator 104 as shown in Figures 1 and 3. The electrode region 114 includes the substrate 110 supporting a plurality of electrodes 116 of the electrode array 102 as best shown in Figure 2. Although four electrodes 116 are illustrated in Figure 2, it should be appreciated that the electrode array 102 may have any number of electrodes 116 such as one, two, three, four, or more electrodes 116. The interconnect 112 and electrode 114 regions may be as small or as large as desired and/or required. Typically, the size and/or configuration of the electrode region 114 depends on the shape of the area where the electrode array 102 is to be implanted and/or the number of electrodes 116 of the electrode array 102. Other factors may affect the size and/or configuration of the electrode region 114. As mentioned above, the stimulator 104 and the electrode array 102 are connected by the connector 108. The connector 108 is connected to the electrodes 116 of the electrode array 102 via a conductive wire, for example, embedded in the substrate 110.

The substrate 110 of the electrode array 102 is formed from a nonconductive material. In an embodiment, the nonconductive material is both an electrically insulating material and a biocompatible material. In an embodiment, the nonconductive material has a resistivity of at least 1×10¹¹ ohm·m. Non-limiting examples of biocompatible nonconductive materials for the substrate 110 include silicones, fluoropolymers (such as polytetrafluoroethylene), oxides, pre-stretched elastomers, poly(p-xylene), polyimides, polyurethanes, and combinations thereof. In a particular embodiment, the nonconductive material is a silicone or combination of silicones. In an alternative embodiment, the nonconductive material could be a silicone(s) in combination with another biocompatible nonconductive material. Use of biocompatible nonconductive materials for the substrate 110 other than silicone is also contemplated.

The substrate 110 may have any suitable configuration and/or size. In the embodiment shown in Figure 2, the substrate 110 has a substantially rectangular configuration and has a length L₁₁₀ and width W₁₁₀. Alternatively, the substrate 110 may have an oval or other suitable configuration. In instances where the substrate 110 has a substantially rectangular configuration, the corners of the substrate 110 are typically rounded. The length L₁₁₀ of the substrate 110 is at least partially the length of the area where the electrode array 102 is to be implanted and/or a length suitable to support a desired number of electrodes 116. For example, the length L₁₁₀ of the substrate 110 may be longer for electrode arrays 102 having six electrodes 116 compared to electrode arrays 102 having two electrodes 116. In another example, the length L₁₁₀ of the substrate 110 may be longer for electrode arrays 102 to be implanted into a long canal (such as the cochlea 12) compared to electrode arrays 102 to be implanted or used in a more open area. For the cochlear prosthesis 100, the length L₁₁₀ of the substrate 110 may be from about 90 to about 110 mm. Additionally, the substrate 110 may have any width W₁₁₀ so long as the electrode array 102 can be properly implanted and/or support a desired arrangement of the electrodes 116. For the cochlear prosthesis 100, the width W₁₁₀ is typically less than 1 mm. Substrates 110 that are longer, shorter, wider, or narrower than the ranges provided above are also contemplated.

As mentioned above, the substrate 110 includes the interconnect region 112 that allows for electrical connection of the electrode array 102 to the stimulator 104. As shown in Figures 3-6, the substrate 110 defines the aperture 118 at the interconnect region 112. In the embodiment shown in Figure 3, the substrate 110 defines a bonding pad 120 at the interconnect region 112, and the bonding pad 120 defines the aperture 118. In the illustrated embodiment, four bonding pads 120 are shown, with each bonding pad 120 defining an aperture 118. Although four bonding pads are shown in Figure 3, it should be appreciated that the substrate 110 may define more or less bonding pads 120. The aperture 118 is configured to receive the connector 108 for electrically connecting the electrode array 102 to the stimulator 104.

In the embodiment shown, each bonding pad 120 defines the aperture 118 adapted to receive a respective connector 108 for establishing an electrical connection between a particular electrode 116 of the electrode array 102 and the stimulator 104. As shown, each bonding pad 120 defines a single aperture 118 configured to receive a connector 108 for electrically connecting the plurality of (e.g., four as shown in Figure 3) electrodes 116 to the stimulator 104. Alternatively, each bonding pad 120 could define two or more apertures 118. In another alternative configuration, the substrate 118 could define a single bonding pad 120, and the single bonding pad 120 could define all of the apertures 118. It should further be appreciated that the substrate 110 can support any number of electrodes 116, and the interconnect region 112 can define any number of apertures 118 with each aperture 118 adapted to receive a connector 108 to electrically connect a respective one of the electrodes 116 of the electrode array 102 to the stimulator 104.

As also shown in Figure 3, each of the bonding pads 120 are spaced from one another. Additionally, each of the apertures 118 are spaced from one another. The apertures 118 define a pitch P₁₁₈ between adjacent apertures 118, which is measured from the center of one aperture 118 to the center of an adjacent aperture 118. The pitch P₁₁₈ between adjacent apertures 118 can have any suitable length. In an embodiment, the pitch P₁₁₈ between adjacent apertures 118 is from about 50 to about 1500 µm. In another embodiment, the pitch P₁₁₈ between adjacent apertures 118 is from about 80 to about 120 µm. In still another embodiment, the pitch P₁₁₈ between adjacent apertures 118 is from about 90 to about 110 µm.

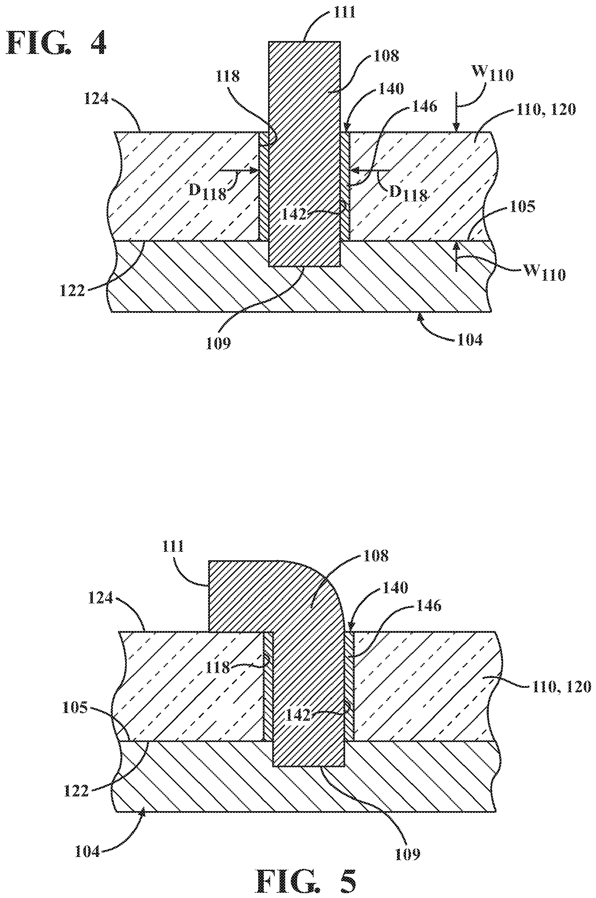

As best shown in Figures 4-6, the aperture(s) 118 may have any suitable configuration, such as a rounded configuration, a square configuration, etc. In an embodiment, the aperture(s) 118 has a rounded configuration with a diameter D₁₁₈ of from about 20 to about 330 µm. In another embodiment, the aperture(s) 118 has a rounded configuration with a diameter D₁₁₈ of from about 30 to about 200 µm. In yet another embodiment, the aperture(s) 118 has a rounded configuration with a diameter D₁₁₈ of from about 30 to about 150 µm. It still another embodiment, the aperture(s) 118 has a rounded configuration with a diameter D₁₁₈ of from about 40 to about 70 µm. It should be appreciated that the size of the aperture 118 depends, at least in part, on the size of the connector 108 to be partially received in the aperture 118. In the embodiments described below, the aperture(s) 118 is larger than the connector 108 to enable at least a portion of the connector 108 can be readily placed and/or received within the apertures 118 and to form a space 140 between the connector 108 and the substrate 110. The space 140 is described in further detail below.

The aperture(s) 118 is defined at least partially through the width W₁₁₀ of the substrate 110 (or bonding pad 120). In an embodiment, and as shown in Figures 4 and 5, the aperture(s) 118 is defined through the entire width W₁₁₀ of the substrate 110 (or bonding pad 120). For example, the substrate 110 has opposing first 122 and second 124 sides, and the aperture(s) 118 is defined through both of the sides 122, 124 of the substrate 110.

With reference to Figures 3 and 4, the connector 108 has first 109 and second 111 ends with the first end 109 attached to the stimulator 104 and the second end 111 spaced from the stimulator 104. In the illustrated embodiments, the first end 109 of the connector 108 is embedded in and thus extends into the stimulator 104. Alternatively, the first end 109 of the connector 108 could be attached to a surface 105 of the stimulator 104, such as welded or the like. The connector 108 extends from the stimulator 104, through the first side 122 of the substrate 110, through the aperture 118, and through the second side 124 of the substrate 110. The second end 111 of the connector 108 protrudes above or beyond the second side 124 of the substrate 110. As best shown in Figure 4, when the connector 108 extends through the aperture 118, the space 140 is defined between the connector 108 and the substrate 110. More particularly, the space 140 is defined at least between a side 142 of the connector 108 and the substrate 110.

The stimulating device 100 further includes a conductive elastic ink 146 disposed at least partially in the space 140 to secure the connector 108 in the aperture 118. The conductive elastic ink 146 may be the same material used to form the electrodes 116 of the electrode array 102. In this embodiment, the conductive elastic ink 146 may be disposed within the space 140 during formation of the electrode array 102. Alternatively, and due at least in part to the elastic nature of the conductive elastic ink (which is described in further detail below), the electrodes 116 formed from the conductive elastic ink could be stretched over the connector 108 to secure the connector 108 within the aperture 118. In another embodiment, such as shown in Figure 5, the second end 111 of the connector 108 is bent to form a hook that engages the second side 124 of the substrate 110 to secure the connector 108 in the aperture 118.

The connector 108 is connected to the electrodes 116 of the electrode array 102 via a conductive wire, for example, embedded in the substrate 110. The conductive elastic ink securing the connector 108 in the aperture 118 also operates to establish or complete the electrical connection between the electrode array 102 and the stimulator 104. Additional layers of the conductive ink may also be added to further secure the electrical connection.

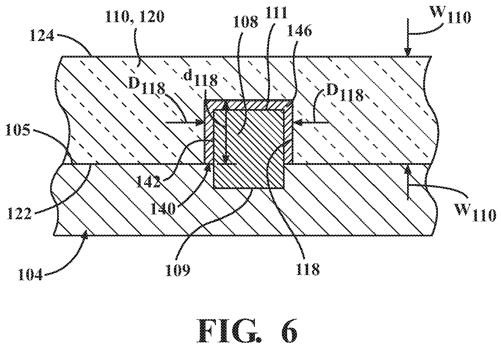

In another embodiment, and as shown in Figure 6, the aperture(s) 118 is defined partially through the width W₁₁₀ of the substrate 110 (or bonding pad 124). For example, the aperture(s) 118 is defined through the first side 122 of the substrate 110, but does not extend through the entire width W₁₁₀ of the substrate 110 (or bonding pad 120). The aperture 118 may have any suitable depth d₁₁₈ so long as the aperture 118 is not formed through the entire width W₁₁₀ of the substrate 110. In an embodiment, the depth d₁₁₈ of the aperture 118 is at least about 5 µm. It should be appreciated that the depth d₁₁₈ could be smaller than 5 µm, rendering the aperture 118 as practically flush with the first side 122 of the substrate 110. As shown in Figure 6, just a portion of the connector 108 including the second end 111 is received in the aperture 118.

When the portion of the connector 108 is disposed within the aperture 118, the space 140 is defined between the connector 108 and the substrate 110. In this embodiment, the space 140 is defined at least between a side 142 of the connector 108 and the substrate 110. The space 140 may also be defined between the second end 111 of the connector 108 and the substrate 110. The conductive elastic ink 146 is disposed in the space 140 to secure the connector 108 in the aperture 118. The conductive elastic ink 146 may be disposed within the space 140 during formation of the electrode array 102. The conductive elastic ink 146 disposed within the space 140, which is in electrical connection with the electrodes 116 of the electrode array 102, also operates to establish or complete the electrical connection between the electrode array 102 and the stimulator 104.

The aperture(s) 118 may be formed during formation of the substrate 110, or subsequent to formation of the substrate 110 utilizing any suitable subtractive manufacturing technique. Alternatively, the aperture(s) 118 may be formed as the connector 108 is disposed (such as punched) through the substrate 110. In this alternative embodiment, the aperture(s) 118 has substantially the same configuration as the connector 108.

The substrate 110 may be formed by depositing the nonconductive material into a mold utilizing any suitable additive manufacturing process or technique. In an embodiment, the nonconductive material, which may take the form of a nonconductive ink, is deposited utilizing a printing technique, such as pressure-driven extrusion printing. This technique may be performed utilizing a suitable pressure-driven extrusion printer, such as an nScrypt^{®} 3Dn printer available from nScrypt Inc. (Orlando, FL). Alternatively, the substrate 110 may be formed using other manufacturing processes, such as by chemical and/or physical deposition processes.

The nonconductive ink used for forming the substrate includes the nonconductive material and a curing agent. In an embodiment, the nonconductive ink further includes a solvent such as, but not limited to, xylene, toluene, ligroin, mineral spirits, chlorinated hydrocarbons, and combinations thereof. The nonconductive may further include one or more additives.

In an embodiment, the substrate 110 includes one or more three-dimensional (3D) features, such as channel(s) or other structure(s) to aid in surgical placement or implantation of the electrode array 102 into the organism 10. Additionally, the first 122 and/or second 124 side of the substrate 110 may be roughened to improved adhesion of subsequently formed components, such as the electrodes 116, of the electrode array 102. Other features may include a wavy surface contour to enhance the ability of the electrode array 102 to stretch without adversely affecting the performance of conductive features (e.g., the electrodes 116) and/or the substrate 110 may be pre-stretched to enhance elasticity of the electrode array 102. One or more of these features may be obtained during the 3D printing to form the substrate 110.

Referring again to Figure 2, the electrode array 102 includes the plurality of electrodes 116 supported by the substrate 110. As previously mentioned, the electrodes 116 are supported by the substrate 110 at the electrode region 114. The electrodes 116 may have any desirable arrangement on the substrate 110. For example, the electrodes 116 may be arranged in a single row, in a plurality of rows, according to a pattern, randomly, or combinations thereof. Additionally, the electrodes 116 are spaced from one another. By virtue of the nonconductive substrate material surrounding the electrodes 116, the electrodes 116 remain electrically isolated from one another.

The electrodes 116 are formed from a conductive elastic ink. The conductive elastic ink comprises conductive particles and a vehicle including silicone, such as polydimethylsiloxane, which imparts elasticity to the conductive elastic ink enabling the electrode array 102 to bend while the electrodes 116 remain electrically conductive. The silicone is typically used with a curing agent. In an embodiment, the conductive elastic ink further includes a siloxane and optionally one or more solvents, such as a traditional solvent, e.g., toluene, heptane, etc. The siloxane functions to reduce the viscosity of the conductive elastic ink and tends to evaporate slowly compared to traditional solvents, thereby maintaining ink consistency during printing. Non-limiting examples of suitable siloxanes for the conductive ink include cyclic siloxanes (such as hexamethylcyclotrisiloxane, octamethylcyclotetrasilocane, etc.), linear siloxanes (such as hexamethylcyclotrisoloxane, octamethyltrisoloxane, etc.), and combinations thereof. One or more additives (such as surfactants, etc.) may also be used in the conductive elastic ink.

The conductive particles of the conductive elastic ink are biocompatible and typically have a resistivity of less than 1×10⁻⁷ ohm·m. Non-limiting examples of conductive particles for the ink include medical grade platinum particles, silver particles, copper particles, gold particles, chromium particles, titanium particles, iridium particles, stainless steel particles, conductive polymer particles, carbon nanotubes, and combinations thereof. Typically, the conductive particles have an effective particle diameter of less than 1 µm and may be referred to as nanoparticles. In an embodiment, the particles have an effective particle diameter of from about 50 nm to about 1 µm. Alternatively, the particles could have an effective particle diameter up to about 10 µm. In a particular embodiment, the conductive elastic ink includes platinum particles as the conductive particles, and the conductive elastic ink may be referred to as a platinum ink. In another embodiment, the platinum particles may be optimized by including conductive particles of varying shapes, sizes, and/or concentrations to obtain superior electrical properties such as charge storage capacity, impedance, etc.

The conductive elastic ink may be formed by combining the conducive particles, the silicone, the siloxane, and optionally one or more additives. Combining may be accomplished, for example, by mixing the conductive particles, the silicone, the siloxane, and optionally the additives with sonication, planetary centrifugal mixing, roll-to-roll milling, or the like. Other methods of combining the components of the conductive elastic ink known in the art are also contemplated.

The electrodes 116 are formed by depositing the conductive elastic ink onto the substrate 110 utilizing any suitable additive manufacturing process, such as by pressure-driven extrusion printing. In an embodiment, the conductive elastic ink is deposited utilizing a printer, such as the nScrypt° printer used to deposit the nonconductive material to form the substrate 110 mentioned above.

The electrode array 102 of the present disclosure is advantageously flexible and elastic such that the electrode array 102 can be used for stimulating devices, such as neuroprosthetic devices, to be implanted or used in curved areas or spaces. In an embodiment, the electrode array 102 is stretchable for up to about 140% elongation while remaining electrically conductive. The stretchability of the electrode array 102 is measured using a tensile test where the electrode array 102 is immersed in saline for 10 days at 37°C and removed to undergo a percent (%) elongation at 1 mm/s followed by a 1-minute hold before returning to the relaxed state. The electrode array 102 was stretched until no electrical resistance could be recorded. The electrode array 102 also passes the 15 degree flex test (where the array 102 was flexed 15 degrees at 2 Hz around a 2mm rod while undergoing a 0.03N force for 100,000 cycles), a 90 degree bend test (where the array 102 was bent 90 degrees around a 5mm rod at 1 mm/s for 10 cycles), and a 360 degrees torsion test (where the array 102 was twisted 360 degrees forward before reversing 360 degrees at 1 mm/s for 50 cycles). Based, at least on these results, the electrode array 102 complies with European Standard EN 45502 parts 1 and 2 and with American National Standard ANSI/AAMI C186:2017 for cochlear implant systems.

Additionally, the electrodes 116 of the electrode array 102 exhibit a cathodic charge storage capacity of from about 30 to about 200 mC/cm², and an electrochemical impedance spectroscopy (EIS) of from about 100 to about 5000Ω at 1 kHz for a line thickness of from about 25 to about 300 µm. The electrodes 116 (formed from the first and second conductive inks including at least conductive particles and silicone) exhibits a lower polarization compared to Platinum foil electrodes. This lower voltage potential indicates that a higher charge density may be inputted without causing tissue damage to the organism.

The present disclosure has been described in an illustrative manner, and it is to be understood that the terminology which has been used is intended to be in the nature of words of description rather than of limitation. It is now apparent to those skilled in the art that many modifications and variations of the present disclosure are possible in light of the above teachings. For example, the electrode array 102 could have electrodes 116 stacked in a vertical direction. In this example, the side 122 of the substrate 110 could serve as a surface to build the additional electrodes 116 in the vertical direction. Additionally, several layers of electrodes 116 separated by substrate layers 110 may be formed, such as by pressure-driven extrusion printing, on top of one another to create a high density of electrodes to minimize the footprint of the electrode array 102 while maximizing functionality. It is, therefore, to be understood that the present disclosure may be practiced otherwise than as specifically described.

## Claims

1. A stimulating device comprising:
a stimulator (104) for generating electrical signals;
an electrode array (102) for transmitting the electrical signals to a target and said electrode array including a substrate (110) defining an aperture (118) and at least one electrode (116) supported by said substrate;
a connector (108) extending from said stimulator and said connector disposed at least partially in said aperture of said substrate and defining a space (140) between said connector and said substrate, with said connector configured to establish an electrical connection between said stimulator and said electrode array; and
a conductive elastic ink (146) disposed at least partially within said space to secure said connector in said aperture.

2. The stimulating device as set forth in claim 1 wherein said substrate has an electrode region (114) and an interconnect region (112) with said at least one electrode supported by said substrate at said electrode region, and said substrate defining said aperture at said interconnect region.

3. The stimulating device as set forth in claim 2 wherein said substrate has a width and said aperture is defined at least partially through said width of said substrate.

4. The stimulating device as set forth in claim 3 wherein said connector is further defined as a connector pin, and said connector pin is disposed at least partially in said aperture and secured to said substrate by said conductive elastic ink.

5. The stimulating device as set forth in claim 3 wherein said aperture is defined entirely through said width of said substrate.

6. The stimulating device as set forth in claim 5 wherein said connector further includes opposing first and second ends (109, 111) with said first end (109) attached to said stimulator and said second end (111) defining a hook abutting said substrate to further secure said connector to said substrate.

7. The stimulating device as set forth in claim 1 wherein said aperture is one of a plurality of apertures defined in said substrate with said plurality of apertures defining a pitch (P118) between adjacent apertures of from about 50 µm to about 1500 µm.

8. The stimulating device as set forth in any preceding claim, wherein said at least one electrode is formed from said conductive elastic ink.

9. The stimulating device as set forth in any preceding claim, wherein said conductive elastic ink comprises conductive particles and silicone with said silicone imparting elasticity to said electrode array.

10. The stimulating device as set forth in claim 9 wherein said conductive particles are chosen from platinum particles, silver particles, copper particles, gold particles, chromium particles, titanium particles, iridium particles, stainless steel particles, conductive polymer particles, carbon nanotubes, and combinations thereof.

11. The stimulating device as set forth in claim 9 wherein said conductive elastic ink further includes a siloxane chosen from cyclic siloxanes, linear siloxanes, and combinations thereof.

12. The stimulating device as set forth in claim 9 wherein said conductive particles have an effective particle diameter of less than 1 µm.

13. The stimulating device as set forth in any preceding claim, wherein said at least one electrode of said electrode array is stretchable up to about 140% elongation while remaining electrically conductive.

14. The stimulating device as set forth in any preceding claim, wherein said stimulating device is further defined as a neuroprosthetic device.

## Patentansprüche

1. Stimulationsvorrichtung, die Folgendes umfasst:
einen Stimulator (104) zum Erzeugen von elektrischen Signalen,
eine Elektrodengruppierung (102) zum Übermitteln der elektrischen Signale an ein Ziel, und wobei die Elektrodengruppierung ein Substrat (110), das eine Öffnung (118) definiert, und mindestens eine Elektrode (116), die durch das Substrat getragen wird, einschließt,
einen Verbinder (108), der sich von dem Stimulator aus erstreckt, und wobei der Verbinder zumindest teilweise in der Öffnung des Substrats angeordnet ist und einen Raum (140) zwischen dem Verbinder und dem Substrat definiert, wobei der Verbinder dafür konfiguriert ist, eine elektrische Verbindung zwischen dem Stimulator und der Elektrodengruppierung herzustellen, und
eine leitfähige elastische Farbe (146), die zumindest teilweise innerhalb des Raumes angeordnet ist, um den Verbinder in der Öffnung zu befestigen.

2. Stimulationsvorrichtung nach Anspruch 1, wobei das Substrat einen Elektrodenbereich (114) und einen Verbindungsbereich (112) aufweist, wobei die mindestens eine Elektrode durch das Substrat bei dem Elektrodenbereich getragen wird und das Substrat die Öffnung bei dem Verbindungsbereich definiert.

3. Stimulationsvorrichtung nach Anspruch 2, wobei das Substrat eine Breite aufweist und die Öffnung zumindest teilweise durch die Breite des Substrats hindurch definiert ist.

4. Stimulationsvorrichtung nach Anspruch 3, wobei der Verbinder ferner als ein Verbinderstift definiert ist und der Verbinderstift zumindest teilweise in der Öffnung angeordnet und durch die leitfähige elastische Farbe an dem Substrat befestigt ist.

5. Stimulationsvorrichtung nach Anspruch 3, wobei die Öffnung vollständig durch die Breite des Substrats hindurch definiert ist.

6. Stimulationsvorrichtung nach Anspruch 5, wobei der Verbinder ferner ein erstes und ein entgegengesetztes zweites Ende (109, 111) einschließt, wobei das erste Ende (109) an dem Stimulator befestigt ist und das zweite Ende (111) einen Haken definiert, der an das Substrat anstößt, um den Verbinder weiter an dem Substrat zu befestigen.

7. Stimulationsvorrichtung nach Anspruch 1, wobei die Öffnung eine von einer Vielzahl von Öffnungen ist, die in dem Substrat definiert sind, wobei die Vielzahl von Öffnungen einen Abstand (P118) zwischen benachbarten Öffnungen von etwa 50 µm bis etwa 1500 µm definiert.

8. Stimulationsvorrichtung nach einem der vorhergehenden Ansprüche, wobei die mindestens eine Elektrode aus der leitfähigen elastischen Farbe gebildet ist.

9. Stimulationsvorrichtung nach einem der vorhergehenden Ansprüche, wobei die leitfähige elastische Farbe leitfähige Teilchen und Silikon umfasst, wobei das Silikon der Elektrodengruppierung Elastizität verleiht.

10. Stimulationsvorrichtung nach Anspruch 9, wobei die leitfähigen Teilchen aus Platinteilchen, Silberteilchen, Kupferteilchen, Goldteilchen, Chromteilchen, Titanteilchen, Iridiumteilchen, rostfreien Stahlteilchen, leitfähigen Polymerteilchen, Kohlenstoff-Nanoröhrchen und Kombinationen derselben ausgewählt sind.

11. Stimulationsvorrichtung nach Anspruch 9, wobei die leitfähige elastische Farbe ferner ein Siloxan einschließt, das aus zyklischen Siloxanen, linearen Siloxanen und Kombinationen derselben ausgewählt ist.

12. Stimulationsvorrichtung nach Anspruch 9, wobei die leitfähigen Teilchen einen wirksamen Teilchendurchmesser von weniger als 1 µm aufweisen.

13. Stimulationsvorrichtung nach einem der vorhergehenden Ansprüche, wobei die mindestens eine Elektrode der Elektrodengruppierung bis zu etwa 140 % Längung dehnbar ist, während sie elektrisch leitfähig bleibt.

14. Stimulationsvorrichtung nach einem der vorhergehenden Ansprüche, wobei die Stimulationsvorrichtung ferner als eine neuroprothetische Vorrichtung definiert ist.

## Revendications

1. Dispositif de stimulation comprenant :
un stimulateur (104) pour générer des signaux électriques ;
un réseau d'électrodes (102) pour transmettre les signaux électriques à une cible et ledit réseau d'électrodes incluant un substrat (110) définissant une ouverture (118) et au moins une électrode (116) supportée par ledit substrat ;
un connecteur (108) s'étendant dudit stimulateur et ledit connecteur disposé au moins partiellement dans ladite ouverture dudit substrat et définissant un espace (140) entre ledit connecteur et ledit substrat, avec ledit connecteur configuré pour établir une connexion électrique entre ledit stimulateur et ledit réseau d'électrodes ; et
une encre élastique conductrice (146) disposée au moins partiellement à l'intérieur dudit espace pour fixer ledit connecteur dans ladite ouverture.

2. Dispositif de stimulation selon la revendication 1, dans lequel ledit substrat présente une région d'électrodes (114) et une région d'interconnexion (112) avec ladite au moins une électrode supportée par ledit substrat sur ladite région d'électrodes, et ledit substrat définissant ladite ouverture sur ladite région d'interconnexion.

3. Dispositif de stimulation selon la revendication 2, dans lequel ledit substrat présente une largeur et ladite ouverture est définie au moins partiellement à travers ladite largeur dudit substrat.

4. Dispositif de stimulation selon la revendication 3, dans lequel ledit connecteur est en outre défini en tant qu'une broche de connecteur, et ladite broche de connecteur est disposée au moins partiellement dans ladite ouverture et fixée sur ledit substrat par ladite encre élastique conductrice.

5. Dispositif de stimulation selon la revendication 3, dans lequel ladite ouverture est définie entièrement à travers ladite largeur dudit substrat.

6. Dispositif de stimulation selon la revendication 5, dans lequel ledit connecteur inclut en outre des première et seconde extrémités opposées (109, 111) avec ladite première extrémité (109) fixée sur ledit stimulateur et ladite seconde extrémité (111) définissant un crochet adjacent audit substrat pour fixer encore plus ledit connecteur sur ledit substrat.

7. Dispositif de stimulation selon la revendication 1, dans lequel ladite ouverture est l'une d'une pluralité d'ouvertures définies dans ledit substrat avec ladite pluralité d'ouvertures définissant un pas (P118) entre des ouvertures adjacentes d'environ 50 um à environ 1500 um.

8. Dispositif de stimulation selon l'une quelconque revendication précédente, dans lequel ladite au moins une électrode est formée à partir de ladite encre élastique conductrice.

9. Dispositif de stimulation selon l'une quelconque revendication précédente, dans lequel ladite encre élastique conductrice comprend des particules conductrices et de la silicone avec ladite silicone conférant de l'élasticité audit réseau d'électrodes.

10. Dispositif de stimulation selon la revendication 9, dans lequel lesdites particules conductrices sont choisies parmi des particules de platine, particules d'argent, particules de cuivre, particules d'or, particules de chrome, particules de titane, particules d'iridium, particules d'acier inoxydable, particules de polymère conducteur, nanotubes de carbone et des combinaisons de ceux-ci.

11. Dispositif de stimulation selon la revendication 9, dans lequel ladite encre élastique conductrice inclut en outre un siloxane choisi parmi les siloxanes cycliques, les siloxanes linéaires et des combinaisons de ceux-ci.

12. Dispositif de stimulation selon la revendication 9, dans lequel lesdites particules conductrices présentent un diamètre de particules effectif inférieur à 1 um.

13. Dispositif de stimulation selon l'une quelconque revendication précédente, dans lequel ladite au moins une électrode dudit réseau d'électrodes est étirable jusqu'à environ 140 % d'allongement tout en restant conductrice électrique.

14. Dispositif de stimulation selon l'une quelconque revendication précédente, dans lequel ledit dispositif de stimulation est en outre défini en tant qu'un dispositif neuroprothétique.
